# EUROPEAN PATENT APPLICATION

(11) **EP 0 811 696 A2**
(43) Date of publication of application: **10.12.1997**
(21) Application number: 97303900.1
(22) Date of filing: 06.06.1997
(51) Int. Cl.: C12Q 1/68

(54) **Method to obtain nucleotide sequence using randomly primed amplification**

(30) Priority: 06.06.1996 US 19234; 01.11.1996 US 29928; 01.11.1996 US 29929
(71) Applicant: SMITHKLINE BEECHAM CORPORATION, Philadelphia Pennsylvania 19103 (US); SMITHKLINE BEECHAM PLC, Brentford, Middlesex TW8 9EP (GB)
(72) Inventor: Guth, Sabine, SmithKline Beecham Pharm., Collegeville, PA 19426 (US); Prescott Catherine D., SmithKline Beecham Pharm., Collegeville, PA 19426 (US)
(74) Representative: Giddings, Peter John, Dr.

(57) **Abstract**

The present invention provides a novel method for obtaining additional sequence data from partial gene fragments independent of probing a library of genomic clones.

SEQUENCE LISTING

(1) GENERAL INFORMATION

(i) APPLICANT: Guth, Sabine
Prescott, Catherine

(ii) TITLE OF THE INVENTION: Method to Obtain Nucleotide
Sequence Using Randomly Primed Application

(iii) NUMBER OF SEQUENCES: 11

(iv) CORRESPONDENCE ADDRESS:
(A) ADDRESSEE: SmithKline Beecham Corporation
(B) STREET: 709 Swedeland Road
(C) CITY: King of Prussia
(D) STATE: PA
(E) COUNTRY: USA
(F) ZIP: 19406-0939

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER:
(B) FILING DATE: 06-JUN-1997
(C) CLASSIFICATION:

(vii) PRIOR APPLICATION DATA:
(A) APPLICATION NUMBER: 60/019,234

(B) FILING DATE: 06-JUN-1996

(A) APPLICATION NUMBER: 60/029,928
(B) FILING DATE: 01-NOV-1996

(A) APPLICATION NUMBER: 60/029,929
(B) FILING DATE: 01-NOV-1996

(viii) ATTORNEY/AGENT INFORMATION:
(A) NAME: Gimmi, Edward R
(B) REGISTRATION NUMBER: 38,891
(C) REFERENCE/DOCKET NUMBER: P50584

(ix) TELECOMMUNICATION INFORMATION:
(A) TELEPHONE: 610-270-4478
(B) TELEFAX: 610-270-5090
(C) TELEX:

(2) INFORMATION FOR SEQ ID NO: 1:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 1:

CGTATTGCTC TTTTAATCTT GTTTC 25

(2) INFORMATION FOR SEQ ID NO: 2:

(i) SEQUENCE CHARACTERISTICS:
(A) LENGTH: 85 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 2:

GTTTTCATTC CCTACCCTAT TTGCAAACAG ACTCCATTCT GAAGCCCTAC GCAACTCCCC 60
AATTACAAAA GTAAGGGATG GGATA 85

(2) INFORMATION FOR SEQ ID NO: 3:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 3:

GTTTTCATTC CCTACCCTAT CC 22

(2) INFORMATION FOR SEQ ID NO: 4:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 4:

CGTATTGCTC TTTTAATCTT GTTTC 25

(2) INFORMATION FOR SEQ ID NO: 5:

(i) SEQUENCE CHARACTERISTICS:
(A) LENGTH: 213 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 5:

GTTTTCATTC CCTACCCTAT CCTTACTTAA TCTTTTTATT AAAAAGAATG CAGATTTTCA 60
GAGAATATAT AAAAAAGGTC ATTCTGTAGC CAACAGACAA TTTGTTGTAT ACACTTGTAA 120
TAATAAAGAA ATAGACCATT TTCGCTTAGG TATTAGTGTT TCTAAAAAAC TAGGTAATGC 180
AGTGTTAAGA ACAAGATTAA AAGAGCAATA CGT 213

(2) INFORMATION FOR SEQ ID NO: 6:

(i) SEQUENCE CHARACTERISTICS:
(A) LENGTH: 215 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 6:

ACGATAAATA ACAGTATGTT ATTGGAAAAA GTCTACCGAA TTAAAAAGAA TGCAGATTTT 60
CAGAGAATAT ATAAAAAAGG TCATTCTGTA GCCAACAGAC AATTTGTTGT ATACACTTGT 120
AATAATAAAG AAATAGACCA TTTTCGCTTA GGTATTAGTG TTTCTAAAAA ACTAGGTAAT 180
GCAGTGTTAA GAACAAGATT AAAAGAGCAA TACGT 215

(2) INFORMATION FOR SEQ ID NO:7:

(i) SEQUENCE CHARACTERISTICS:
(A) LENGTH: 170 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:7:

GAAAATTTCA AAGTACATAA GTCGCATATA TTGGCCAAAG ATTGTAATAG CAAGACAGCC 60
AGCTAAAGAT ATGACGACTT TACAAATACA GAATAGTCTT GAGCACGTAC TTAAAATTGC 120
CAAAGTTTTT AATAAAAAGA TTAAGTAAGG ATAGGGTAGG GAATGAAAAC 170

(2) INFORMATION FOR SEQ ID NO:8:

(i) SEQUENCE CHARACTERISTICS:
(A) LENGTH: 354 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO:8:

ATGTTATTGG AAAAAGCTTA CCGAATTAAA AAGAATGCAG ATTTTCAGAG AATATATAAA 60
AAAGGTCATT CTGTAGCCAA CAGACAATTT GTTGTATACA CTTGTAATAA TAAAGAAATA 120

GACCATTTTC GCTTAGGTAT TAGTGTTTCT AAAAAACTAG GTAATGCAGT GTTAAGAAAC 180
AAGATTAAAA GAGCAATACG TGAAAATTTC AAAGTACATA AGTCGCATAT ATTGGCCAAA 240
GATATTATTG TAATAGCAAG ACAGCCAGCT AAAGATATGA CGACTTTACA AATACAGAAT 300
AGTCTTGAGC ACGTACTTAA AATTGCCAAA GTTTTTAATA AAAAGATTAA GTAA 354

(2) INFORMATION FOR SEQ ID NO: 9:

(i) SEQUENCE CHARACTERISTICS:
(A) LENGTH: 117 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 9:

Met Leu Leu Glu Lys Val Tyr Arg Ile Lys Lys Asn Ala Asp Phe Gly
1               5                   10                  15
Arg Ile Tyr Lys Lys Gly His Ser Val Ala Asn Arg Gln Phe Val Val
            20                  25                  30
Tyr Thr Cys Asn Asn Lys Glu Ile Asp His Phe Arg Leu Gly Ile Ser
        35                  40                  45
Val Ser Lys Lys Leu Gly Asn Ala Val Leu Arg Asn Lys Ile Lys Arg
    50                  55                  60
Ala Ile Arg Glu Asn Phe Lys Val His Lys Ser His Ile Leu Ala Lys
65                  70                  75                  80
Asp Ile Ile Val Ile Ala Arg Gln Pro Ala Lys Asp Met Thr Thr Leu
                85                  90                  95
Gln Ile Gln Asn Ser Leu Glu His Val Leu Lys Ile Ala Lys Val Phe
            100                 105                 110
Asn Lys Lys Ile Lys
        115

(2) INFORMATION FOR SEQ ID NO: 10:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 10:

ATGTTATTGG AAAAAGCTTA CCG 23

(2) INFORMATION FOR SEQ ID NO: 11:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: Genomic DNA

(xi) SEQUENCE DESCRIPTION: SEQ ID NO: 11:

CGGGATCCTT ATTACTTAAT CTTTTTATTA AAAACTTTGG C 41

## Description

### RELATED APPLICATIONS

This application claims benefit of U.S. Provisional Application Number 60/019,234, filed June 6, 1996, U.S. Provisional Application Number 60/029,928, filed November 1, 1996, and U.S. Provisional Application Number 60/029,929, filed November 1, 1996.

### BACKGROUND OF THE INVENTION

PCR is a well known method for amplifying polynucleotide sequences (Saiki et al., *Nature, 324:* 163-166 (1986)). While many variations of PCR and other forms of nucleic acid amplification have been disclosed, none has satisfactorily fulfilled the need for a rapid and simple method to obtain unknown sequences from a clone that is incomplete using polynucleotides obtained from an organism without using conventional approaches, such as *in situ* colony hybridization. The present invention provides a rapid method to obtain such unknown sequences.

### SUMMARY OF THE INVENTION

The invention provides a method for obtaining an unknown polynucleotide sequence which is contiguous to a known polynucleotide sequence comprising the steps of amplifying an unknown sequence using a first primer, and amplifying the products of a the amplification of the first amplification step using random primers and a second primer.

Also provided is a method for obtaining an unknown polynucleotide sequence contiguous to a known polynucleotide sequence comprising the steps of selecting a first primer in a known sequence flanking unknown sequences either upstream or downstream of the known sequence, such as, for example, downstream of the stop codon, selecting a second primer in proximity to the end of the known sequence from which the sequence is to be extended, amplifying an unknown sequence using the first primer, and amplifying the products of a the first amplification step using random primers and a second primer to obtain amplified products of unknown sequence. For example, if the missing sequence is upstream or downstream of the known sequence the second primer will be chosen at the end comprising the known sequence.

Further provided is a method for obtaining an unknown polynucleotide sequence contiguous to a known polynucleotide sequence comprising the steps of selecting a first primer flanking unknown sequences either upstream or downstream of the known sequence, selecting a second primer near the end of a known sequence, amplifying an unknown sequence using the first primer, and amplifying the products of a the first amplification step using random primers and a second primer. "Near the end" or "close to the end" as used herein means at the terminal nucleotide position of the template, a few nucleotides from this terminus, e..g, 2, 3, 4, 5, 6, 7 8, 9, 10, 20, or 30, or the closest position to the terminus on which a useful primer can be annealed or hybridized to prime an amplification reaction. An example of a second primer "near the end" of a known sequence is provided in the Examples below.

The invention also provides methods wherein the second primer is labeled.

The invention also provides methods wherein the random primers are hexanucleotides or longer sequences.

The invention also provides methods whereby the first or second amplifying step comprises about 10 reaction cycles.

The invention provides methods whereby an amplifying step comprises PCR or such step amplifies an RNAseP sequence.

### BRIEF DESCRIPTION OF THE FIGURES

**Figure 1** shows a schematic diagram of a specific embodiment of the first amplification step.

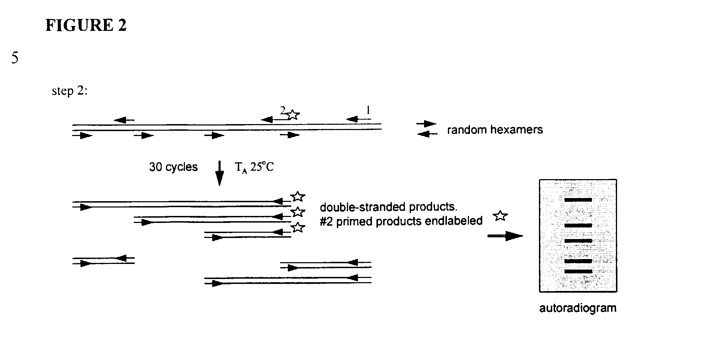

**Figure 2** shows a schematic diagram of a specific embodiment of the second amplification step.

### DETAILED DESCRIPTION OF THE INVENTION

The present invention provides a novel, rapid and simple method for obtaining additional sequence data from partial gene fragments independent of the conventional methods of probing a genomic library.

Random sequencing of any genomic library followed by sequence homology searching with another sequence set results in the identification of a desired fragment. Where a conceptual or deduced translation product of the polynucleotide region from the desired fragment shows significant homology to the C-terminal portion of a homologous protein but the N-terminal portion is missing the method of the invention can be used to obtain the missing N-terminal portion.

Certain embodiments of this method comprise a two step amplification reaction using two different reverse primers complementary to a known sequence (the "known sequence" or "known sequence fragment") at any position on the known sequence, as well as random primer, e.g., hexamer (hexanucleotide) primers or longer, for example, those provided commercially (e.g., Gibco) or those made using known methods. These primers are used to obtain an extended sequence of a known sequence fragment, such as, for example, a complete 3' or 5' polynucleotide sequence. To obtain such sequence, a selected sequence fragment of an organism's nucleic acid, such as a genomic DNA or cDNA, is partially digested with restriction enzyme to linearize it for use as a template for amplification. Circularized template may also be used but linear template is preferred. A first primer (herein "primer #1" or "first primer") is annealed to flanking unknown sequences either upstream or downstream of the known sequence fragment and a second primer (herein "primer #2" or "second primer") is positioned close to the end of the known sequence region of the fragment (e.g., proximal to the terminus of the known sequence, preferably the primer starts at the terminus and extends along its length). It is preferred in the methods of the invention that the amplifying steps use PCR (Saiki et al., *Nature, 324*: 163-166 (1986)). Using this method, this amplification of the desired sequence favored the binding of random primers to that sequence in the second step.

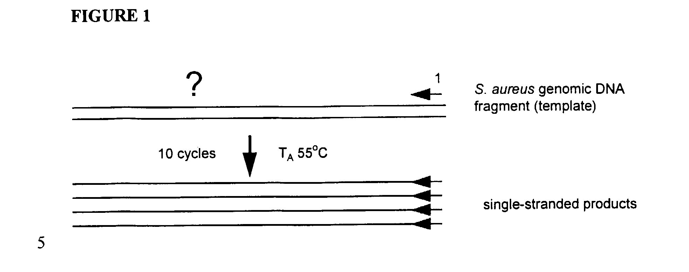

In the first step, the reverse strand encoding the C-terminus and upstream the unknown N-terminal sequence of the protein was amplified using primer #1, resulting in single stranded products. The amplification temperature is preferably between about 25°C and 75°C, more preferably between about 50°C and 70°C, and most preferably about 55°C. The cycle number is preferably between about 1 and 100 cycles, more preferably between about 5 and 20 cycles, and most preferably about 10 cycles. A schematic of a specific example method of the invention is depicted in Figure 1. This schematic in no way limits the scope of the invention but is merely provided to exemplify certain aspects of the invention. The question mark indicates the region of unknown sequence.

In the second step, primer #2, preferably labeled, and random primers, preferably pentamers, hexamers or heptamers, are added and the annealing temperature was dropped to allow the random primers to anneal to the DNA. The annealing temperature may be those known in the art for annealing for amplification reactions, particularly PCR reactions, and is preferably about 25°C. Labeling of the primer is preferably end-labeling using any known method to label a polynucleotide primer. Preferred labeling methods include, for example, ³²P-end-labeling, fluorescent labeling, or colorimetic labeling, especially those labels typically employed for nucleic acid labeling.

The amplification reaction is, in any given reaction, predicted to generate many different fragments. However, the major products are those primed by primer #2 and a random primer. These products are of particular interest as they are expected to generate the missing portion of the sequence (e.g., the C- or N-terminal half of the protein) and they may be simply monitored since they are labeled. After separation of the amplification products using known methods, such as, for example, by gel electrophoresis and exposure to film, a limited number of labeled bands are identifiable, such as through visual or automated inspection of an autoradiogram. These fragments can subsequently be cloned into a suitable vector, e.g., pUC19 or pBR322, and sequenced, such as using well known sequencing methods.

The invention may be used to amplify and determine unknown sequence from any organism as well as from mixtures of polynucleotide sequences from several organisms or chemical synthesis reactions, for example, from virus, bacteriophage, eukaryotes, and prokaryotes, such as eubacteria, archaebacteria, or a polynucleotide synthesizer. Reverse transcription may be used prior to performing the amplification reactions so that reverse transcriptase copies of RNAs encoded by unknown sequences may be amplified and the unknown sequences obtained.

As used herein, "primer(s)" refers to relatively short polynucleotides or oligonucleotides, preferably sequences of about 5 to 50 nucleotides in length. Primers, such as single-stranded DNA oligonucleotides, often are synthesized by chemical methods, such as those implemented on automated oligonucleotide synthesizers. However, primers can be made by a variety of other methods, including *in vitro* recombinant DNA-mediated techniques and by expression of DNAs in cells and organisms. Initially, chemically synthesized DNAs typically are obtained without a 5' phosphate. The 5' ends of such oligonucleotides are not substrates for phosphodiester bond formation by ligation reactions that employ DNA ligases typically used to form recombinant DNA molecules. The 3' end of chemically synthesized primer generally has a free hydroxyl group and, in the presence of a ligase, such as T4 DNA ligase, readily will form a phosphodiester bond with a 5' phosphate of another polynucleotide, such as another oligonucleotide. As is well known, this reaction can be prevented selectively, where desired, by removing the 5' phosphates of the other polynucleotide(s) prior to ligation.

"Plasmid(s)" generally are designated herein by a lower case p preceded and/or followed by capital letters and/or numbers, in accordance with standard naming conventions that are familiar to those of skill in the art. Starting plasmids disclosed herein are either commercially available, publicly available on an unrestricted basis, or can be constructed from available plasmids by routine application of well known, published procedures. Many plasmids and other cloning and expression vectors that can be used in accordance with the present invention are well known and readily available to those of skill in the art. Moreover, those of skill readily may construct any number of other plasmids suitable for use in the invention. The properties, construction and use of such plasmids, as well as other vectors, in the present invention will be readily apparent to those of skill from the present disclosure.

As used herein "polynucleotides(s)" or "sequence(s)" generally refer to any polyribonucleotide or polydeoxribonucleotide, which may be unmodified RNA or DNA or modified RNA or DNA. Thus, for instance, polynucleotides as used herein refers to, among others, single-and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded, or a mixture of single- and double-stranded regions. In addition, polynucleotide as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the term polynucleotide or sequences includes DNAs or RNAs as described above that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including simple and complex cells, *inter alia.*

### Examples

The present invention is further described by the following examples. The examples are provided solely to illustrate the invention by reference to specific embodiments. These exemplifications, while illustrating certain specific aspects of the invention, do not portray the limitations or circumscribe the scope of the disclosed invention.

All examples were carried out using standard techniques, which are well known and routine to those of skill in the art, except where otherwise described in detail. Routine molecular biology techniques of the following examples can be carried out as described in standard laboratory manuals, such as Sambrook et al., *MOLECULAR CLONING: A LABORATORY MANUAL,* 2nd Ed.; Cold Spring Harbor Laboratory Press, Cold Spring Harbor, N.Y. (1989).

All parts or amounts set out in the following examples are by weight, unless otherwise specified.

Unless otherwise stated size separation of fragments in the examples below was carried out using standard techniques of agarose and polyacrylamide gel electrophoresis ("PAGE") in Sambrook et al., *MOLECULAR CLONING: A LABORATORY MANUAL,* 2nd Ed., Cold Spring Harbor Laboratory Press, Cold Spring Harbor, N.Y. (1989) and numerous other references such as, for instance, by Goeddel et al., *Nucleic Acids Res. 8:* 4057 (1980).

### Example 1 Obtaining Missing Sequences of a S. aureus Genomic Clone

Random sequencing of a S. *aureus* genomic library followed by sequence homology searching with the *B. subtilis* P-protein sequence resulted in the identification of a 324 base pairs (herein "bp") fragment. The first 193 nucleotides of this fragment showed significant homology to the C-terminal half of the *B. subtilis* P-protein and other prokaryotic RNase P proteins. The N-terminal domain of the putative *S*. *aureus* RNase P protein was missing. Putative *S*. *aureus* RNase P protein is shown in Table 1. S. *aureus* versus *B. subtilis:* 58.7% similarity, 34.9% identity.

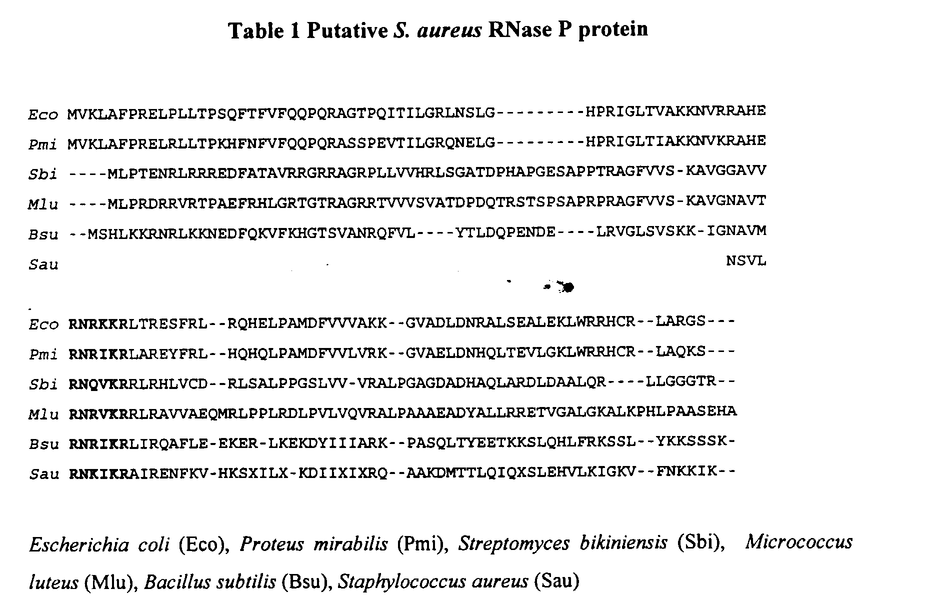

An embodiment of the two step PCR reaction of the invention was used with two different reverse primers complementary to the known sequence in position 15-39 and 194-215 respectively (shown below) as well as random hexamer primers (Gibco) was used to obtain the complete 5' sequence of the S. *aureus spp* gene. S. *aureus* genomic DNA partially digested with Hind III or Pst I served as a template. Primer #1 (position 194-215, Table 2) annealed just downstream of the stop codon TAA and primer #2 (position 15-39, Table 2) close to the end of the known sequence.

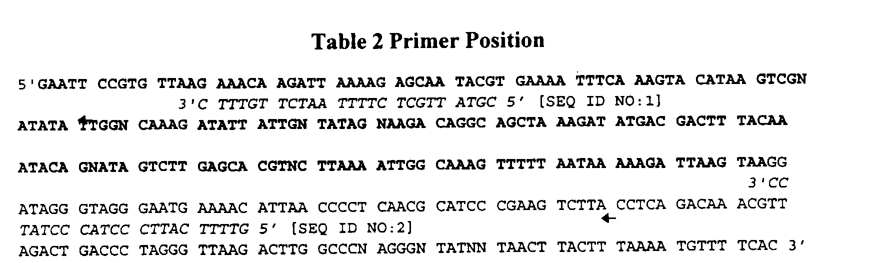

In the first step, the reverse strand encoding the C-terminus and upstream the unknown N-terminal sequence of the protein was amplified using primer #1, resulting in single stranded products. This amplification of the desired sequence favored the binding of random primers to that sequence in the next step.

In the second step, ³²P-end-labeled primer #2 and random hexamers were added and the annealing temperature was dropped to 25°C to allow the short random primers to anneal to the DNA.

The PCR was predicted to generate many different fragments, however the major products should be those primed by primer #2 and a random primer (see below). These products were of most interest as they should encode the N-terminal half of the protein and they could be monitored since they would be radiolabeled. After separation of the PCR products by gel electrophoresis and exposure to film, a limited number of radiolabeled bands should be visible on the autoradiogram. These fragments can subsequently be cloned into a suitable vector (e.g. pUC19) and sequenced.

### Example 2 PCR with random primers

Two primers were designed (custom synthesized by Gibco BRL) with the following sequences: primer #1 (22mer) 5' GTTTTCATTCCCTACCCTATCC 3' [SEQ ID NO:3] and primer #2 (25mer) 5' CGTATTGCTCTTTTAATCTTGTTTC 3' [SEQ ID NO:4].

The reaction mix (100 ml) for the first step of amplification comprised:
- 71 ml: sterile ddH₂O
- 10 ml: 10x ThermoPol reaction buffer (NEB)
- 2 ml: 10mM dNTP mix (dATP, dGTP, dCTP, dTTP)
- 5 ml: primer #1 at 100 ng/ml
- 1 ml: *S*. *aureus* genomic DNA at 200 ng/ml (HindIII digested)
- 10 ml: random primers (Gibco BRL #48190-011) at 10 ng/ml
- 1 ml: Vent Polymerase (NEB) at 2U/ml

All the reaction components were added to a thin-walled 500 ml tube (Perkin Elmer GeneAmp tubes) and overlayed with 2 drops of mineral oil (Sigma) to prevent evaporation during PCR.

The reaction mix was incubated at 95°C for 3 minutes to denature the DNA. The first PCR step was carried out in a Perkin Elmer DNA Thermocycler 480 using 10 cycles of 45 sec at 95°C, 45 sec at 55°C and 1 minutes at 72°C. After that 5 ml of radiolabeled primer #2 mix (15 ml primer#2 at 100ng/ ml, I ml [g-32P]ATP-end-labeled primer #2, 2ml 10x ThermoPol buffer) were added to the reaction mix and PCR was continued for 60 cycles with a decreased annealing temperature: 45 sec at 95°C, 45 sec at 25°C and minutes at 72°C.

A sample of the products was analyzed by gel electrophoresis, stained with ethidium bromide and the DNA visualized under UV light. The gel was then exposed to film to visualize the radiolabeled bands.

The products were cloned into pUC19 and sequenced. The additional sequence information completed the *spp* gene. Accordingly the entire gene could be recovered by PCR in the following manner:

The 5' half of the *spp* gene was amplified in a two step PCR reaction as described above. The PCR products were cleaned using a QIAquick PCR purification column and recovered in 50 ml water. The sample was concentrated to a final volume of 25 ml under vacuum in a Speedvac® SC110 (Savant). 20ml were loaded onto a 1.6 mm, 8% polyacrylamide gel containing 7M urea and analyzed by gel electrophoresis. The gel was stained with ethidium bromide (1 mg/ml in H₂O) to visualize the DNA and photographed. The gel was then dried and exposed to film.

There were several defined bands visible both on the ethidium bromide stained gel as well as on the autoradiogram. They ranged in size between 50 bp to over 2000 bp, some bands being more intense than others. The most prominent bands were approximately 50bp, 150bp, 400bp and 800bp in size.

### Example 3 Cloning and sequencing of the PCR fragments

The PCR fragments were blunt end cloned into Smal cut pUC19. Ligations of 100 ng vector DNA with 1.0 ml and 1.5 ml of PCR products were performed at 16°C overnight. *E. coli* XL1 blue cells (alpha-complementing) were transformed by electroporation and plated onto selective plates containing IPTG and x-gal (for blue/white screening).

Transformation of these cells resulted in approximately 8.5% white colonies in the presence of PCR products, whereas only 4.6% white colonies could be found in the control ligation without insert. White colonies had lost the ability to form the b-galactosidase holoenzyme as a result of a reading frame disruption in the *lacZ'* gene and were no longer able to cleave x-gal. The reading frame was either disrupted by an insert (in the presence of PCR products) or exonuclease activity of the restriction enzyme preparation, destroying the Sma I site of the vector by cleaving terminal nucleotides and resulting in frame shifts after ligation.

18 white colonies were picked and grown up as 2 ml cultures to prepare plasmids. A sample of each plasmid was linearized with AccI restriction endonuclease (cuts in the multi cloning site) and run on a 1% TAE agarose gel alongside the original pUC19.

7 plasmids released a short fragment upon restriction digest. As there is only one AccI site in pUC19, these recombinant plasmids must contain an insert with a restriction site for this enzyme. 3 plasmids appeared larger than pUC19 but did not release a fragment. The other 8 plasmids were of the same size as pUC19 and did not appear to have an insert.

The 10 plasmids containing an insert were sequenced both with the forward and reverse pUC/M13 sequencing primers.

5 of the 6 plasmids that released a fragment after AccI digest harboured an 213bp insert that started with the primer #2 sequence and the known 14bp followed by a sequence of 174 nucleotides that was common to all of the plasmids (sequence 1, Table 3). In one of the 6 plasmids the insert (sequence 2, Table 3) was slightly different: the first 137 nucleotides were in common with sequence 1 (dotted line, Table 3), however the insert sequence extended beyond this and deviated from sequence 1 (see Table 3).

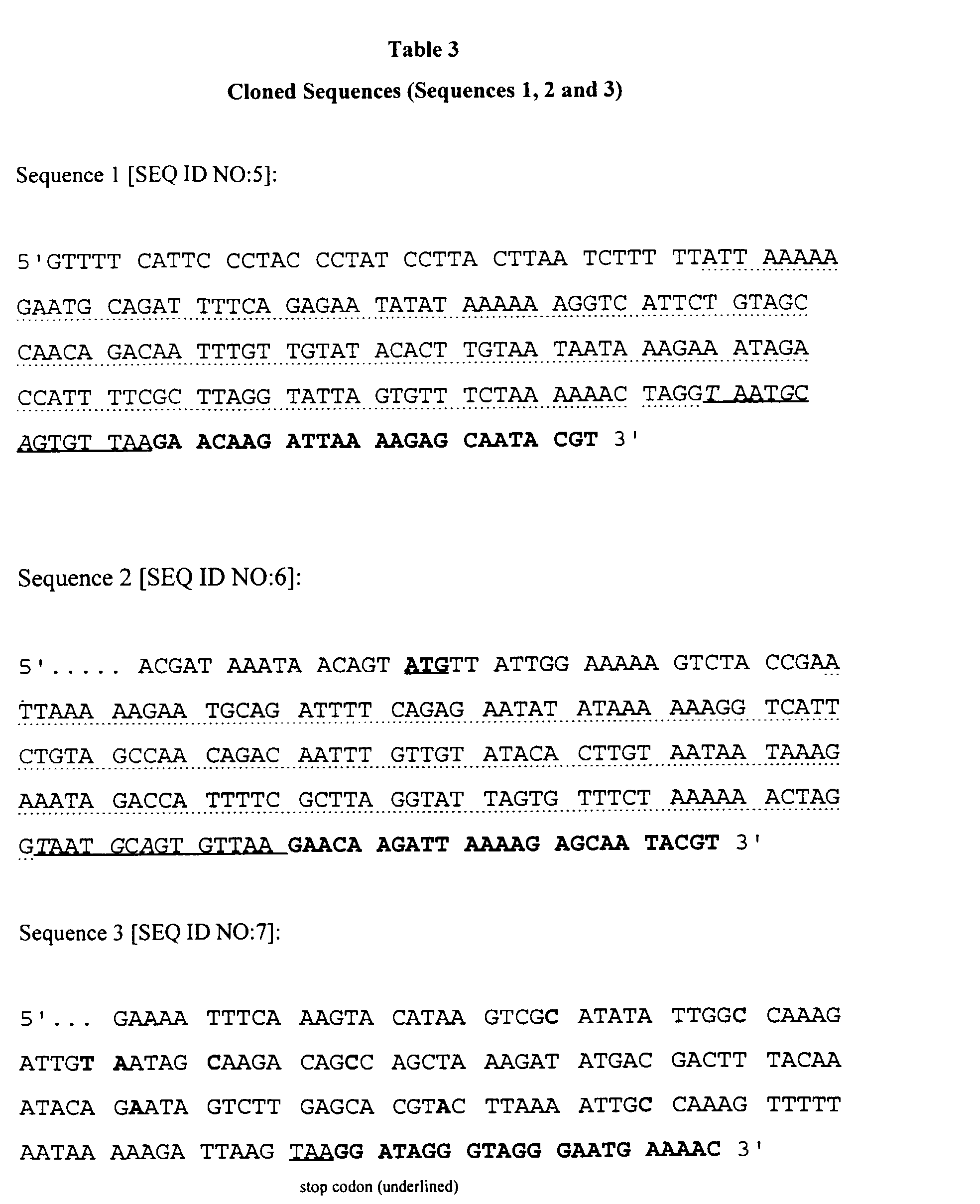

In Table 3, the primer #2 sequence is shown in bold and known 14 bases underlined (3 changes to known sequence in italics).

The predicted translation products of sequence 1 and 2 (see Table 3) showed significant homology to *B. subtilis* P-protein and indicated that these inserts contain the full sequence information for the 5' domain of the *spp* gene.

In 2 plasmids the insert started with the sequence of primer #1 followed by 3' half of the *spp* gene (sequence 3, Table 3). This sequence was already known but the new sequence showed 9 base changes (shown in bold in Table 3) in comparison to the original sequence data obtained by random sequencing of the *S. aureus* library.

2 plasmids contained inserts with different sequences that are a result of unspecific priming during PCR.

### Example 4 Analysis of the sequence data

The newly obtained sequence data was drawn together and predicted to represent the complete sequence of the *spp* structural gene. The reading frame was already known from the partial sequence of the 3' domain. According to this reading frame the start codon ATG could be identified 174bp upstream of primer #2 sequence (see above, Table 3). The total length of the gene is 354bD (Table 4).

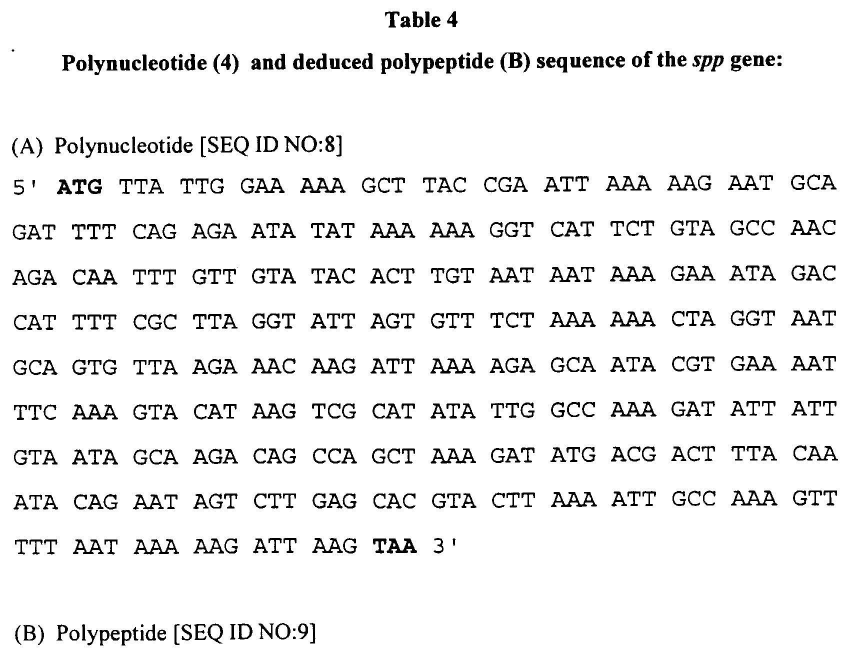

MLLEK VYRIK KNADF GRIYK KGHSV ANRQF VVYTC NNKEI DHFRL
GISVS KKLGN AVLRN KIKRA IRENF KVHKS HILAK DIIVI ARQPA
KDMTT LQIQN SLEHV LKIAK VFNKK IK

The predicted translation product of the *spp* gene (SP protein) is 117 amino acids [SEQ ID NO:9]: This translated sequence was aligned with the published amino acid sequences of the RNase P protein from 5 different prokaryotes as shown in Table 5.

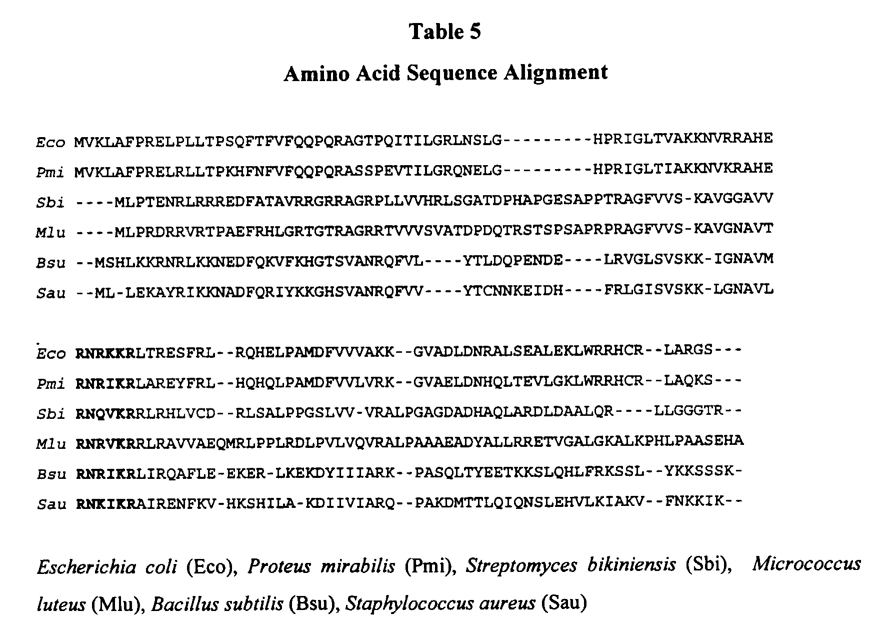

The *S. aureus* SP protein sequence matched the *B. subtilis* P-protein sequence well, revealing significant homology to the *B. subtilis* protein.

### Example 5 PCR of the spp gene

The *S*. *aureus spp* gene was amplified via PCR using the following DNA primers:

forward primer (23mer):
5' ATGTTATTGGAAAAAGCTTACCG 3' [SEQ ID NO:10]

reverse primer (41mer):
5'CGGGATCCTTATTACTTAATCTTTTATTAAAAACTTTGGC 3' [SEQ ID
NO:11]

The reverse primer included a BamHI restriction site (shown in bold) attached to the 5' end for directional cloning into pMalc2.

Genomic *S*. *aureus* DNA partially digested with Hind III restriction endonuclease served as a template.

The 100 µl PCR comprised:
- 77 µl: sterile ddH₂O
- 10 µl: 10x ThermoPol Buffer (NEB)
- 2 µl: 10mM dNTP-mix (dATP, dGTP, dTTP, dCTP)
- 5 µl: forward primer at 100 ng/µl
- 5 µl: reverse primer at 100 ng/µl
- 1 µl: *S*. *aureus* genomic DNA at 200 ng/µl
- 1 µl: Vent DNA Polymerase (NEB)

All the reaction components were added to a thin-walled 500 µl tube (Perkin Elmer GeneAmp tubes) and overlayed with 2 drops of mineral oil (Sigma) to prevent evaporation during PCR.

The reaction mix was incubated at 95°C for 3 min to denature the DNA. The PCR amplification was carried out in a Perkin Elmer DNA Thermocycler 480 using 30 cycles of 45 sec at 95°C, 45 sec at 55°C and 1 min at 72°C.

The PCR product was cleaned using the QIAquick PCR Purification Kit to remove unused primers and the mineral oil. A sample was analysed by agarose gel electrophoresis.

The newly obtained sequence data was used to design PCR primers for the amplification of the full length *spp* structural gene.

The PCR resulted in a single band of approximately 360bp with a total DNA yield of 2 µg as determined by spectrophotometrical measurement. The DNA fragment was cleaned using a QIAquick PCR purification column and recovered in 80µl water.

## Claims

1. A method for obtaining an unknown polynucleotide sequence which is contiguous to a known polynucleotide sequence comprising the steps of:
(a) amplifying an unknown sequence using a first primer;
(b) amplifying the products of a the amplification of (a) using random primers and a second primer.

2. A method for obtaining an unknown polynucleotide sequence contiguous to a known polynucleotide sequence comprising the steps of:
(a) selecting a first primer flanking unknown sequences either upstream or downstream of the known sequence;
(b) selecting a second primer near the end of a known sequence;
(c) amplifying an unknown sequence using the first primer;
(d) amplifying the products of a the amplification of (c) using random primers and a second primer to obtain amplified products of unknown sequence.

3. A method for obtaining an unknown polynucleotide sequence contiguous to a known polynucleotide sequence comprising the steps of:
selecting a first primer flanking unknown sequences either upstream or downstream of the known sequence;
selecting a second primer near the end of a known sequence;
(a) amplifying an unknown sequence using the first primer;
(b) amplifying the products of a the amplification of (a) using random primers and a second primer.

4. The method of claim 1 wherein the second primer is labeled.

5. The method of claim 1 wherein the random primers are hexanucleotides.

6. The method of claim 1 whereby the amplifying step (a) comprises about 10 reaction cycles.

7. The method of claim 2 whereby the an amplifying step comprises PCR.

8. The method of claim 2 whereby an amplifying step amplifies an RNAseP sequence.

9. The method of claim 2 wherein the second primer is labeled.

10. The method of claim 2 wherein the random primers are hexanucleotides.

11. The method of claim 2 whereby the amplifying step (a) comprises about 10 reaction cycles.

12. The method of claim 2 whereby the an amplifying step comprises PCR.

13. The method of claim 2 whereby an amplifying step amplifies an RNAseP sequence.

14. The method of claim 3 wherein the second primer is labeled.

15. The method of claim 3 wherein the random primers are hexanucleotides.

16. The method of claim 3 whereby the amplifying step (a) comprises about 10 reaction cycles.

17. The method of claim 3 whereby the an amplifying step comprises PCR.

18. The method of claim 3 whereby an amplifying step amplifies an RNAseP sequence.

19. A method for amplifying RNase P polypeptide using the primers set forth in SEQ ID NOS: 10 or 11.

20. A polynucleotide selected from the group consisting of SEQ ID NOS: 1, 2, 3, 4, 10, and 11.
